# EUROPEAN PATENT APPLICATION

(11) **EP 4 772 515 A1**
(43) Date of publication of application: **08.07.2026**
(21) Application number: 24950303.8
(22) Date of filing: 19.08.2024
(51) Int. Cl.: C07D 519/00, A61K 31/53, A61P 31/14

(54) **PREPARATION METHOD FOR SALT OF CYCLIC CARBONATE NUCLEOSIDE COMPOUND AND CRYSTAL FORM THEREOF**

(30) Priority: 06.08.2024 CN 202411068927
(71) Applicant: Guangdong Chenkang Biotechnology Co. Ltd., Guangzhou, Guangdong 510700 (CN)
(72) Inventor: LIAO, Guochao, Guangzhou, Guangdong 510700 (CN); HAO, Linghua, Guangzhou, Guangdong 510700 (CN); LIAO, Pan, Guangzhou, Guangdong 510700 (CN); ZHANG, Jiangang, Guangzhou, Guangdong 510700 (CN); LIN, Guoming, Guangzhou, Guangdong 510700 (CN)
(74) Representative: Sun, Yiming
(86) International application number: PCT/CN2024/113198
(87) International publication number: WO 2026/031273

(57) **Abstract**

The present invention belongs to the field of pharmaceutical technology and relates to a salt of a cyclic carbonate nucleoside compound, its crystal forms, preparation methods, and applications. The salt of the cyclic carbonate nucleoside compound has the structure shown in Formula I. When Y is hydrobromic acid and n=1, the salt of this nucleoside compound exists as Crystal Form A or Crystal Form B. Crystal Form A has advantages such as good physical and chemical stability, good solid properties, good water solubility, low hygroscopicity, and high oral bioavailability. It can be used to prepare drugs for treating and/or alleviating related diseases caused by viruses (particularly novel coronavirus, feline infectious peritonitis virus, respiratory syncytial virus, porcine epidemic diarrhea virus, feline calicivirus, etc.).

## Description

### Cross-Reference to Related Applications

This application claims priority to Chinese Patent Application No. 2024110689279, titled "A Salt of a Cyclic Carbonate Nucleoside Compound, Its Crystal Forms, and Preparation Methods," filed on August 6, 2024, the entire disclosure of which is incorporated herein by reference in its entirety.

### Technical Field

The present invention belongs to the field of pharmaceutical technology, and particularly relates to a salt of a cyclic carbonate nucleoside compound, its crystal forms, preparation methods, and applications.

### Background Art

The novel coronavirus (SARS-CoV-2), which has a high genetic sequence similarity to the SARS-CoV discovered in 2003, has spread rapidly worldwide since its emergence. This type of virus is not only highly transmissible and mutable but also difficult to prevent through vaccines due to its high replication capability. Currently, there are no effective chemical drugs for Respiratory Syncytial Virus (RSV), which is the leading cause of viral respiratory infection hospitalizations in infants and young children, posing a serious threat to child health. In this context, antiviral drugs, particularly nucleoside analogues targeting viral genetic material (DNA or RNA), have become a key strategy.

Some coronaviruses can also infect various mammals, including cats, pigs, dogs, cattle, horses, and camels. For instance, an estimated 40-80% of cats globally carry feline coronavirus (FCoV), which is divided into two subtypes.

Notably, the feline infectious peritonitis virus (FIPV) type, mutated from feline coronavirus, has an extremely high fatality rate. Similarly, Porcine Epidemic Diarrhea Virus (PEDV) also belongs to the coronavirus family and causes high morbidity and mortality in sows. Furthermore, Feline Calicivirus (FCV) is a highly variable, single-stranded RNA virus that primarily causes acute upper respiratory infections in cats with high incidence rates and is currently considered a possible major cause of feline stomatitis.

Therefore, there is an urgent need to develop nucleoside analogues with stable physical properties and excellent pharmacokinetic characteristics for treating various infectious diseases caused by these viruses.

### Summary of the Invention

In view of the deficiencies in the prior art, the present invention provides a salt of a cyclic carbonate nucleoside compound, its crystal forms, preparation methods, and applications. The salt of the cyclic carbonate nucleoside compound and its crystal forms provided by the present invention exhibit characteristics such as simple preparation, high reproducibility, and good physical and chemical stability.

In a first aspect, the present invention provides a salt of a cyclic carbonate nucleoside compound, having the structure shown in Formula I:

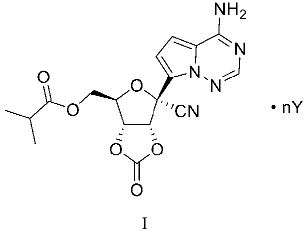

Wherein: Y is hydrochloric acid, sulfuric acid, nitric acid, phosphoric acid, hydrobromic acid, maleic acid, succinic acid, tartaric acid, hydroiodic acid, fumaric acid, benzoic acid, phthalic acid, methanesulfonic acid, benzenesulfonic acid, p-toluenesulfonic acid, camphoric acid, camphorsulfonic acid, salicylic acid, trifluoroacetic acid, acetic acid, perchloric acid, malic acid, formic acid, propionic acid, malonic acid, oxalic acid, naphthalenesulfonic acid, acetylsalicylic acid, aspartic acid, glutamic acid, lactic acid, gluconic acid, ascorbic acid, gallic acid, mandelic acid, sorbic acid, taurine, homotaurine, cinnamic acid, mucic acid, or a combination thereof, preferably hydrobromic acid or hydrochloric acid; n is 0.5-2, preferably 1.

Preferably, when the positively charged hydrogen ion in Y forms a salt with the nitrogen atom at the 3-position of the 4-aminopyrrolo[2,1-fl[1,2,4]triazine heterocycle structure in the free base of the compound of Formula I, said Formula I compound is a compound of Formula I-1, wherein Y-H represents Y minus one H atom.

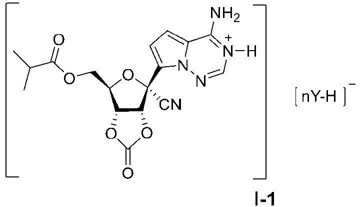

Wherein: n is 0.5-2, preferably 1.

Specifically, when n is 1 and Y is hydrobromic acid in the compound of Formula I-1, the compound is the compound shown in Formula I-1'.

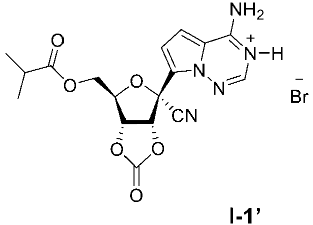

In a second aspect, the present invention provides a crystal of the compound shown in Formula I-1'. Said crystal has Crystal Form A or Crystal Form B.

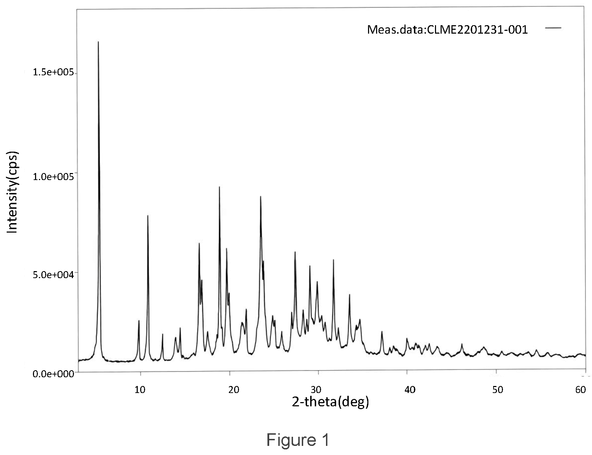

The X-ray powder diffraction pattern of said Crystal Form A has characteristic peaks at at least 3, preferably at least 5, more preferably at least 7 of the following 2θ values: 5.42±0.2°, 10.87±0.2°, 16.56±0.2°, 18.88±0.2°, 21.83±0.2°, 23.81±0.2°, and 27.39±0.2°. In some embodiments, the X-ray powder diffraction pattern of said Crystal Form A also has characteristic peaks at at least 3, preferably at least 5, more preferably at least 8 of the following 2θ values: 25.09±0.2°, 27.02±0.2°, 28.26±0.2°, 29.05±0.2°, 29.84±0.2°, 31.71±0.2°, 33.52±0.2°, and 37.17±0.2°. In some embodiments, the X-ray powder diffraction pattern of said Crystal Form A is as shown in Figure 1.

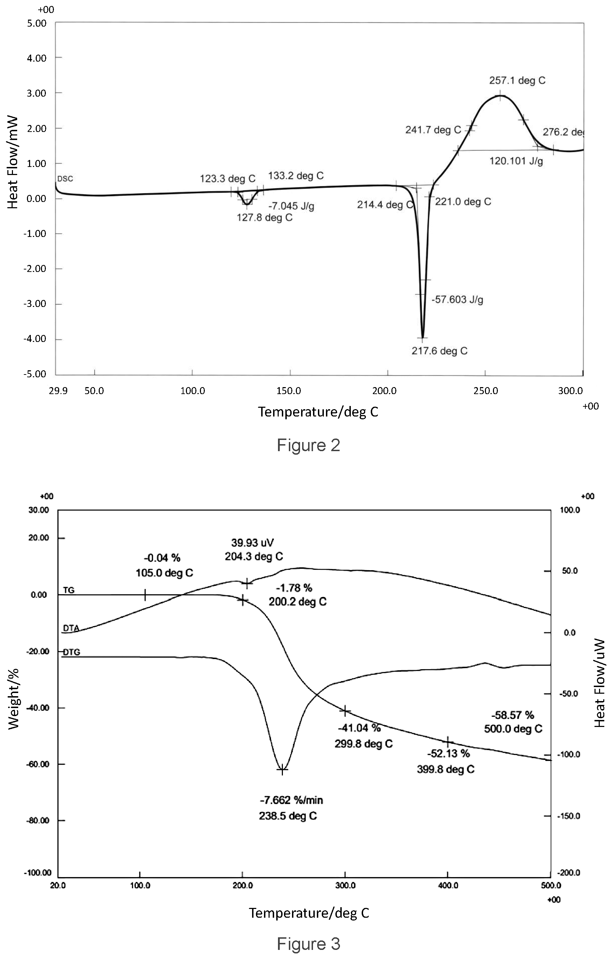

The DSC spectrum of said Crystal Form A has an absorption peak at 217±5°C; in some embodiments, the DSC spectrum of said Crystal Form A is as shown in Figure 2.

The thermogravimetric analysis (TGA) spectrum of said Crystal Form A shows a weight loss of only 0.04% before 105°C, with slow weight loss up to about 180°C followed by rapid weight loss due to sample decomposition, indicating the absence of free water and crystalline solvent in the sample, specifically as shown in Figure 3.

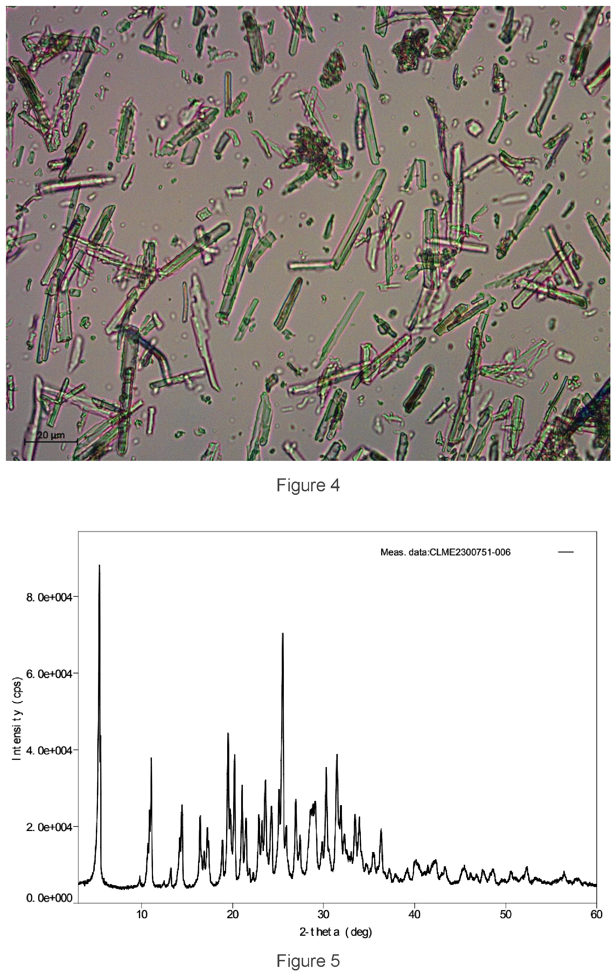

The polarized light microscopy image of said Crystal Form A is shown in Figure 4; the sample consists of needle-like crystals with a particle size range: D10=5 µm; D50=13 µm; D90=27 µm.

The Crystal Form A of the compound shown in Formula I-1' of the present invention has low hygroscopicity. After being placed at 25°C, RH 80% for 24 hours, the moisture increase is below 1%, preferably below 0.2%, more preferably below 0.1%.

The X-ray powder diffraction pattern of said Crystal Form B has characteristic peaks at at least 3, preferably at least 5, more preferably at least 8 of the following 2θ values: 5.33±0.2°, 11.07±0.2°, 19.49±0.2°, 20.19±0.2°, 21.04±0.2°, 25.50±0.2°, 30.31±0.2°, and 31.47±0.2°. In some embodiments, the X-ray powder diffraction pattern of said Crystal Form B also has characteristic peaks at at least 3, preferably at least 5, more preferably at least 8 of the following 2θ values: 14.44±0.2°, 16.41±0.2°, 17.20±0.2°, 19.72±0.2°, 21.46±0.2°, 22.88±0.2°, 23.58±0.2°, and 24.26±0.2°. In some embodiments, the X-ray powder diffraction pattern of said Crystal Form B is substantially as shown in Figure 5.

In a third aspect, the present invention provides a preparation method for a compound as shown in Formula I or Formula I-1', comprising the following steps:

Dissolving a compound of Formula II in Solvent A to obtain Solution A; mixing and stirring an acid or a solution of an acid dissolved in a solvent (Solution B) with said Solution A to obtain a mixed solution; continuing to stir the mixed solution until solid precipitates; filtering; washing the filter cake with an appropriate amount of Solvent A; and drying to obtain the target product.

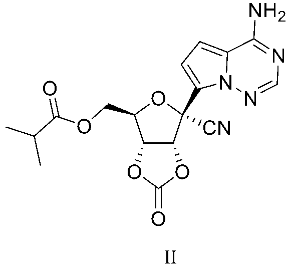

Said compound of Formula II is described in the Chinese patent application CN202210372580.1. This type of compound exhibits significant activity against SARS-CoV-2 virus and feline infectious peritonitis virus, and also has good inhibitory effects against various other RNA viruses. However, at room temperature, this compound is often a viscous gum, insoluble in water, prone to degradation, has poor chemical stability, and poor drugability, posing certain difficulties particularly in pharmaceutical formulation processes. Therefore, finding a solid form of this compound with good water solubility and stable chemical properties is extremely important.

Said acid is hydrochloric acid, sulfuric acid, nitric acid, phosphoric acid, hydrobromic acid, maleic acid, succinic acid, tartaric acid, hydroiodic acid, fumaric acid, benzoic acid, phthalic acid, methanesulfonic acid, benzenesulfonic acid, p-toluenesulfonic acid, camphoric acid, camphorsulfonic acid, salicylic acid, trifluoroacetic acid, acetic acid, perchloric acid, malic acid, formic acid, propionic acid, malonic acid, oxalic acid, naphthalenesulfonic acid, acetylsalicylic acid, aspartic acid, glutamic acid, lactic acid, gluconic acid, ascorbic acid, gallic acid, mandelic acid, sorbic acid, taurine, homotaurine, cinnamic acid, mucic acid, or a combination thereof, preferably hydrobromic acid or hydrochloric acid.

Preferably, in said preparation method, for each 1 g of the compound of Formula II, the amount of Solvent A used is 2-20 mL, preferably 1 g: 10-14 mL; the temperature for preparing the mixed solution and waiting for solid precipitation is -15-50°C, preferably -5-15°C; the molar ratio of the compound of Formula II to acid is 1:0.5-2, preferably 1:1; the amount of Solvent B used to dilute the acid is 1-4 times, preferably 2-3 times, the volume of the acid; the stirring time is 0.5-12 hours, preferably 0.5-4 hours; the amount of Solvent A used to wash the filter cake is 4-20 mL, preferably 8-12 mL. Said Solvent A and Solvent B are each independently selected from ethyl acetate, acetonitrile, acetone, water, and homogeneous mixtures thereof; Solvent A and Solvent B are both preferably ethyl acetate.

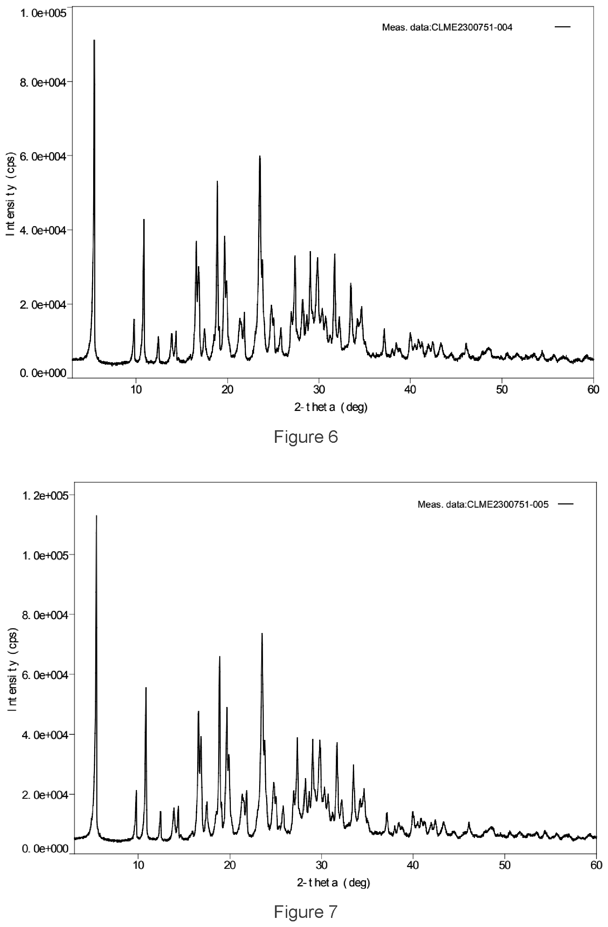

When the salt formation temperature is 38-42°C, the X-ray powder diffraction pattern of the prepared hydrobromide salt is as described in Figure 6, and its 2θ values are substantially consistent with those of Crystal Form A in Figure 1.

The X-ray powder diffraction pattern of Crystal Form A after drying at 80°C for 24 hours is as described in Figure 7, and its 2θ values are substantially consistent with those of Crystal Form A in Figure 1.

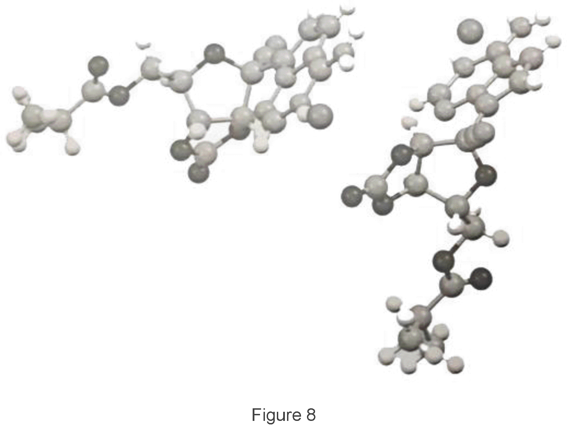

The single crystal diffraction of Crystal Form A is shown in Figure 8, which is consistent with the chemical structure of methyl ((3a*R*,4*R*,6*R*,6a*R*)-6-(4-aminopyrrolo[2,1-*f*][1,2,4]triazin-7-yl)-6-cyano-2-oxotetrahydrofuro[3,4-*d*][1,3]dioxol-4-yl)isobutyrate hydrobromide.

It should be noted that when hydrobromic acid is used as the acid, the above preparation method yields Crystal Form A. As for Crystal Form B of the compound II hydrobromide, its preparation method comprises:
Dissolving the compound of Formula II in Solvent A to obtain Solution A; mixing and stirring hydrobromic acid or a solution of hydrobromic acid dissolved in Solvent B (Solution B) with said Solution A to obtain a mixed solution; continuing to stir the mixed solution, with no solid precipitation; concentrating the mixed solution under reduced pressure to obtain a gum; adding Solvent C to the gum; stirring until a large amount of solid precipitates; filtering; washing the filter cake with an appropriate amount of Solvent C; and drying to obtain the target product.

Preferably, for each 1 g of the compound of Formula II, the amount of Solvent A used is 1-5 mL, preferably 2-3 mL; the amount of Solvent B used to dilute the acid is 1-4 times, preferably 2-3 times, the volume of the acid; the stirring time is 10-60 minutes, preferably 30 minutes; the amount of Solvent C added to the gum is 4-20 mL, preferably 8-12 mL; the amount of Solvent C used to wash the filter cake is 4-20 mL, preferably 8-12 mL. Said Solvent A, Solvent B, and Solvent C are each independently selected from ethyl acetate, acetonitrile, acetone, water, and homogeneous mixtures thereof; Solvent A is preferably acetonitrile, and Solvent C is preferably ethyl acetate.

In a fourth aspect, the present invention provides a pharmaceutical composition comprising the aforementioned salt of the cyclic carbonate nucleoside compound or the crystal of the cyclic carbonate nucleoside compound hydrobromide.

In a fifth aspect, the present invention provides the use of the aforementioned salt of the cyclic carbonate nucleoside compound, crystal of the cyclic carbonate nucleoside compound hydrobromide, or pharmaceutical composition in the treatment of diseases caused by infectious viruses.

Preferably, said infectious viruses include one or more of novel coronavirus, feline infectious peritonitis virus, respiratory syncytial virus, porcine epidemic diarrhea virus, and feline calicivirus.

In a sixth aspect, the present invention provides the use of the aforementioned salt of the cyclic carbonate nucleoside compound, crystal of the cyclic carbonate nucleoside compound hydrobromide, or pharmaceutical composition in the manufacture of a medicament for treating diseases caused by infectious viruses.

Preferably, said infectious viruses include one or more of novel coronavirus, feline infectious peritonitis virus, respiratory syncytial virus, porcine epidemic diarrhea virus, and feline calicivirus.

The present invention provides a salt of a cyclic carbonate nucleoside compound, its crystal forms, preparation methods, and applications. The salt of the cyclic carbonate nucleoside compound has the structure shown in Formula I. When it is the hydrobromide salt, it includes Crystal Form A or Crystal Form B. Crystal Form A has advantages such as good physical and chemical stability, good solid properties, good water solubility, low hygroscopicity, and high oral bioavailability. It can be used to prepare drugs for treating and/or alleviating related diseases caused by viruses (particularly novel coronavirus, feline infectious peritonitis virus, respiratory syncytial virus, porcine epidemic diarrhea virus, feline calicivirus, etc.).

### Brief Description of the Drawings

Figure 1 shows the X-ray powder diffraction pattern of the hydrobromide salt (Crystal Form A) of Compound II.
Figure 2 shows the DSC spectrum of the hydrobromide salt (Crystal Form A) of Compound II.
Figure 3 shows the TGA spectrum of the hydrobromide salt (Crystal Form A) of Compound II.
Figure 4 shows the polarized light microscopy image of the hydrobromide salt (Crystal Form A) of Compound II.
Figure 5 shows the X-ray powder diffraction pattern of the hydrobromide salt (Crystal Form B) of Compound II.
Figure 6 shows the X-ray powder diffraction pattern of the hydrobromide salt of Compound II when the salt formation temperature is 38-42°C.
Figure 7 shows the X-ray powder diffraction pattern of the hydrobromide salt (Crystal Form A) of Compound II after drying at 80°C for 24 hours.
Figure 8 shows the single crystal diffraction pattern of the hydrobromide salt (Crystal Form A) of Compound II.

### Detailed Description of the Embodiments

To make the objectives, technical solutions, and advantages of the present invention clearer, the technical solutions in the present invention will be described clearly and completely below. Obviously, the described embodiments are only a part of the embodiments of the present invention, not all of them.

Based on the embodiments of the present invention, all other embodiments obtained by a person of ordinary skill in the art without making creative efforts shall fall within the protection scope of the present invention.

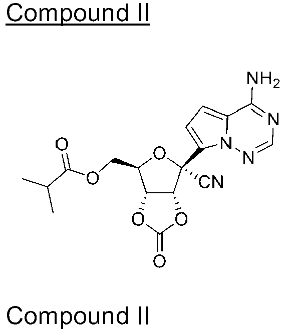

### Reagents and Materials Description

In the embodiments of the present invention, solvents such as ethyl acetate used in the preparation methods are of analytical grade, and unless otherwise specified, the reagents used have not been specially treated.

In the embodiments of the present invention, room temperature refers to 15-25°C.

### General Testing Methods

### 1. X-ray Powder Diffraction (XRPD) Testing Method

Instrument: X-ray polycrystalline diffractometer; Target: Cu-K β (40 kV, 40 mA); Sample-to-detector distance: 30 cm; Scan step width: 0.02°; Scan range: 3°-60°; Scan step time: 0.1 s.

Generally, X-ray powder diffraction angles (2θ values) may have an error within ±0.2°. Therefore, the numerical values of diffraction angles involved in the present invention should be understood to also include values within about ±0.2°. Thus, the present invention encompasses not only crystal forms that completely match the characteristic peaks in the specific X-ray powder diffraction pattern but also crystal forms with an error of about ±0.2° in the characteristic peaks of the specific X-ray powder diffraction pattern.

### 2. DSC Testing Method:

Instrument: Thermo plus EVO2 differential scanning calorimeter; Temperature range: 30-300°C; Heating rate: 10°C/min.

### 3. Hygroscopicity Testing Method:

First, place a stoppered glass weighing bottle in an artificial climate chamber (temperature 25°C±1°C, relative humidity 80%±2%) for 24 h, and accurately weigh the weight of the stoppered glass weighing bottle (m1). Take an appropriate amount of the test sample, spread it evenly in the stoppered glass weighing bottle that has been placed in the artificial climate chamber (temperature 25°C±1°C, relative humidity 80%±2%) for 24 h, and accurately weigh the weight at this time (m2). Leave the weighing bottle open, place it together with the stopper in the artificial climate chamber (temperature 25°C±1°C, relative humidity 80%±2%) for 24 h, then remove and accurately weigh the weight at this time (m3). Calculate the percentage weight gain of the sample.

### 4. Stability Testing Method:

Place the test sample in a suitable clean container, under high temperature (80°C), accelerated (40°C, 75% RH), and long-term (25°C/60% RH) conditions respectively, for 7 days. Sample on day 7, inspect the appearance of the sample visually, and determine the content (purity) by HPLC.

### 5. Solubility Testing Method:

Take about 10 mg of the test sample, place it in a 10 mL volumetric flask, add deionized water to volume, shake vigorously for 30 seconds every 5 minutes at room temperature. After 30 minutes, filter through a 0.45 µm microporous membrane, transfer 2 mL of the filtrate to a 10 mL volumetric flask, add acetonitrile to volume, use as the test sample solution for HPLC analysis, and calculate the solubility of the test sample in pure water using the external standard method.

### Example 1: Preparation of the hydrochloride salt of Compound II

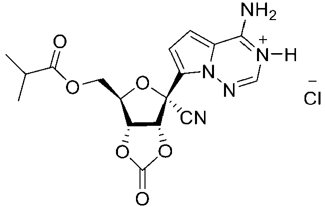

((3a*R*,4*R*,6*R*,6a*R*)-6-(4-aminopyrrolo[2,1-*f*][1,2,4]triazin-7-yl)-6-cyano-2-oxotetrahydrofuro[3,4-*d*][1,3]dioxol-4-yl)isobutyrate (1.0 g, 2.58 mmol) in ethyl acetate (12 mL), add 36% hydrochloric acid (0.26 g, 2.58 mmol) dropwise at room temperature. After addition, continue stirring at room temperature for 30 minutes. Solid precipitates. Filter, wash the filter cake with 8 mL ethyl acetate, and dry the filter cake to obtain the title compound as a white solid (708 mg, yield 64.7%, HPLC purity 99.76%).

¹H-NMR(400 MHz, DMSO-*d*₆): δ 9.87 (s, 1H), 9.20 (s, 1H), 8.27 (s, 1H), 7.43 (d, *J =* 4.7 Hz, 1H), 7.03 (d, *J =* 4.7 Hz, 1H), 5.94 (d, *J =* 7.7 Hz, 1H), 5.51 (dd, *J* = 7.7, 3.7 Hz, 1H), 4.87 (q, *J* = 4.0 Hz, 1H), 4.35 (dd, *J* = 12.3, 4.0 Hz, 1H), 4.26 (dd, *J* = 12.3, 5.2 Hz, 1H), 2.45 (h, *J* = 7.0 Hz, 1H), 1.02 (dd, *J* = 12.6, 7.0 Hz, 6H).

### Example 2: Preparation of the hemisulfate salt of Compound II

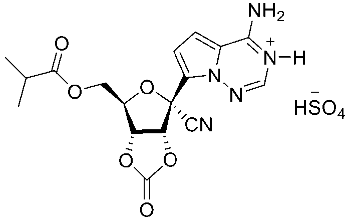

((3a*R*,4*R*,6*R*,6a*R*)-6-(4-aminopyrrolo[2,1-*f*][1,2,4]triazin-7-yl)-6-cyano-2-oxotetrahydrofuro[3,4-*d*][1,3]dioxol-4-yl)isobutyrate (1.0 g, 2.58 mmol) in ethyl acetate (12 mL), add a sulfuric acid solution dropwise at room temperature (98% concentrated sulfuric acid (0.13 g, 1.29 mmol) added to 300 µL water for dilution). After addition, continue stirring at room temperature for 4 h. The reaction mixture becomes gummy. Filter, wash the filter cake with 8 mL ethyl acetate, and dry the filter cake to obtain the title compound as a white solid (870 mg, yield 77.2%, HPLC purity 99.26%).

¹H-NMR(400 MHz, DMSO-*d*₆): δ 9.11 (s, 1H), 8.73 (s, 1H), 8.18 (s, 1H), 7.21 (d, *J =* 4.7 Hz, 1H), 7.00 (d, *J* = 4.7 Hz, 1H), 5.95 (d, *J* = 7.7 Hz, 1H), 5.50 (dd, *J* = 7.7, 3.7 Hz, 1H), 4.86 (q, *J* = 3.9 Hz, 1H), 4.35 (dd, *J* = 12.3, 3.9 Hz, 1H), 4.25 (dd, *J* = 12.3, 5.2 Hz, 1H), 2.44 (h, *J* = 7.0 Hz, 1H), 1.01 (dd, *J* = 13.0, 7.0 Hz, 6H).

### Example 3: Preparation of the phosphate salt of Compound II

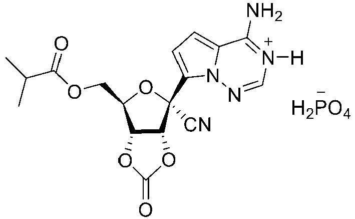

((3a*R*,4*R*,6*R*,6a*R*)-6-(4-aminopyrrolo[2,1-*f*][1,2,4]triazin-7-yl)-6-cyano-2-oxotetrahydrofuro[3,4-*d*][1,3]dioxol-4-yl)isobutyrate (1.0 g, 2.58 mmol) in ethyl acetate (12 mL), add 85% phosphoric acid (0.15 g, 2.58 mmol) dropwise at room temperature. After addition, continue stirring at room temperature for 30 minutes. The reaction mixture becomes compacted. Filter, wash the filter cake with 8 mL ethyl acetate, and dry the filter cake to obtain the title compound as a white solid (934 mg, yield 74.5%, HPLC purity 99.42%).

¹H-NMR(400 MHz, DMSO-*d*₆): δ 8.06 (d, 2H), 7.98 (s, 1H), 6.96 (d, *J* = 4.6 Hz, 1H), 6.91 (d, *J* = 4.6 Hz, 1H), 6.00 (d, *J* = 7.7 Hz, 1H), 5.49 (dd, *J* = 7.7, 3.7 Hz, 1H), 4.82 (q, *J* = 4.0 Hz, 1H), 4.34 (dd, *J* = 12.2, 4.0 Hz, 1H), 4.23 (dd, *J* = 12.2, 5.2 Hz, 1H), 2.44 (p, *J =* 7.0 Hz, 1H), 1.00 (dd, *J* = 13.6, 7.0 Hz, 6H).

### Example 4: Preparation of the nitrate salt of Compound II

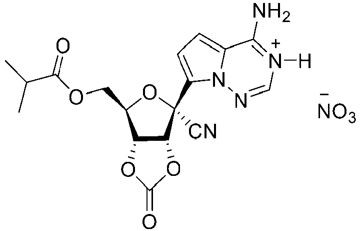

((3a*R*,4*R*,6*R*,6a*R*)-6-(4-aminopyrrolo[2,1-*f*][1,2,4]triazin-7-yl)-6-cyano-2-oxotetrahydrofuro[3,4-*d*][1,3]dioxol-4-yl)isobutyrate (1.0 g, 2.58 mmol) in ethyl acetate (12 mL), add 65% nitric acid (0.25 g, 2.58 mmol) dropwise at room temperature. After addition, continue stirring at room temperature for 30 minutes. Solid precipitates. Filter, wash the filter cake with 8 mL ethyl acetate, and dry the filter cake to obtain the title compound as a white solid (878 mg, yield 75.5%, HPLC purity 99.85%).

¹H-NMR(400 MHz, DMSO-*d*₆): δ 9.49 (s, 1H), 8.98 (s, 1H), 8.25 (s, 1H), 7.29 (d, *J =* 4.7 Hz, 1H), 7.03 (d, *J =* 4.7 Hz, 1H), 5.93 (d, *J =* 7.7 Hz, 1H), 5.51 (dd, *J* = 7.7, 3.7 Hz, 1H), 4.88 (q, *J* = 4.0 Hz, 1H), 4.35 (dd, *J* = 12.3, 4.0 Hz, 1H), 4.26 (dd, *J* = 12.3, 5.1 Hz, 1H), 2.45 (h, *J* = 7.0 Hz, 1H), 1.02 (dd, *J* = 12.7, 7.0 Hz, 6H).

### Example 5: Preparation of the maleate salt of Compound II

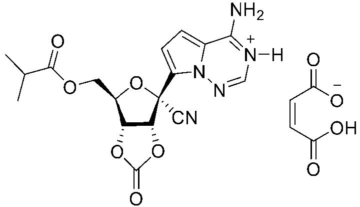

((3a*R*,4*R*,6*R*,6a*R*)-6-(4-aminopyrrolo[2,1-f*]*[1,2,4]triazin-7-yl)-6-cyano-2-oxotetrahydrofuro[3,4-*d*][1,3]dioxol-4-yl)isobutyrate (1.0g, 2.58 mmol) in ethyl acetate (12 mL), add maleic acid (0.30 g, 2.58 mmol) dropwise at room temperature. After addition, continue stirring at room temperature for 3 h. Solid precipitates. Filter, wash the filter cake with 8 mL ethyl acetate, and dry the filter cake to obtain the title compound as a white solid (878 mg, yield 75.5%, HPLC purity 99.60%).

¹H-NMR(400 MHz, DMSO-*d*₆): δ 8.13 (d, 2H), 8.00 (s, 1H), 6.97 (d, *J* = 4.6 Hz, 1H), 6.92 (d, *J* = 4.6 Hz, 1H), 6.27 (s, 2H), 5.99 (d, *J* = 7.7 Hz, 1H), 5.49 (dd, *J =* 7.7, 3.7 Hz, 1H), 4.82 (q, *J* = 4.0 Hz, 1H), 4.34 (dd, *J* = 12.3, 4.0 Hz, 1H), 4.23 (dd, *J =* 12.3, 5.2 Hz, 1H), 2.43 (h, *J =* 7.0 Hz, 1H), 1.01 (dd, *J =* 13.5, 7.0 Hz, 6H).

### Example 6: Preparation of the hydrobromide salt (Crystal Form A) of Compound II

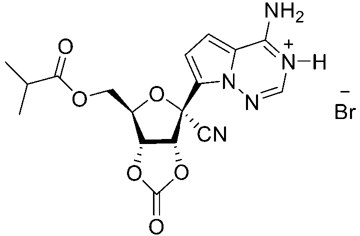

((3a*R*,4*R*,6*R*,6a*R*)-6-(4-aminopyrrolo[2,1-*f*][1,2,4]triazin-7-yl)-6-cyano-2-oxotetrahydrofuro[3,4-*d*][1,3]dioxol-4-yl)isobutyrate (1.0 g, 2.58 mmol) in ethyl acetate (12 mL), add 48% hydrobromic acid (0.43 g, 2.58 mmol) dropwise at room temperature. After addition, continue stirring at room temperature for 30 minutes. Solid precipitates. Filter, wash the filter cake with 8 mL ethyl acetate, and dry the filter cake to obtain a white crystalline solid (1115 mg, yield 92.1%, HPLC purity 99.78%).

¹H-NMR (400 MHz, DMSO-*d*₆): δ 11.34 (s, 1H), 10.31 (s, 1H), 9.54 (s, 1H), 8.44 (s, 1H), 7.64 (d, *J* = 4.8 Hz, 1H), 7.12 (d, *J* = 4.8 Hz, 1H), 6.00 (d, *J* = 7.7 Hz, 1H), 5.59 (dd, *J* = 7.7, 3.6 Hz, 1H), 4.92 (q, *J* = 3.9 Hz, 1H), 4.48 -- 4.21 (m, 2H), 2.47 (h, *J* = 7.0 Hz, 1H), 1.03 (dd, *J* = 12.8, 7.0 Hz, 6H).

¹³C-NMR(100 MHz, DMSO-*d*₆): δ 176.10, 153.33, 149.84, 139.06, 125.53, 115.62, 114.08, 112.86, 109.46, 83.66, 82.63, 80.75, 79.61, 62.64, 33.46, 19.17, 19.07.

MS: m/z 388.1255 [M+1]⁺.

X-ray powder diffraction (XRPD) results showed that the obtained solid was a crystalline form, corresponding to Crystal Form A, as shown in Figure 1.

Differential scanning calorimetry (DSC) analysis of Crystal Form A showed that the obtained solid started to show an endothermic peak at 214.4°C, reaching its peak at 217.6°C, as shown in Figure 2.

Thermogravimetric analysis (TGA) and polarized light microscopy images of Crystal Form A are shown in Figures 3 and 4, respectively.

Single crystal diffraction results of Crystal Form A showed that the positively charged hydrogen ion in hydrobromic acid forms a salt with the nitrogen atom at the 3-position of the pyrrolo[2,1-f][1,2,4]triazine heterocycle structure in Compound II, as shown in Figure 8.

### Example 7: Preparation of the hydrobromide salt (Crystal Form B) of Compound II

((3a*R*,4*R*,6*R*,6a*R*)-6-(4-aminopyrrolo[2,1-*f*][1,2,4]triazin-7-yl)-6-cyano-2-oxotetrahydrofuro[3,4-*d*][1,3]dioxol-4-yl)isobutyrate (1.0 g, 2.58 mmol) in acetonitrile (2.5 mL), add 48% hydrobromic acid (0.43 g, 2.58 mmol) dropwise at room temperature. After addition, continue stirring at room temperature for 30 minutes. Concentrate the reaction mixture under reduced pressure to an oil. Add 12 mL ethyl acetate, stir. A large amount of solid precipitates. Suction filter, wash the filter cake with 8 mL ethyl acetate, and dry the filter cake to obtain a white crystalline solid (1028 mg, yield 85.0%, HPLC purity 99.30%).

X-ray powder diffraction (XRPD) results showed that the obtained solid was a crystalline form, corresponding to Crystal Form B, as shown in Figure 5.

**Example 8:** Preparation of the hydrobromide salt of Compound II at a salt formation temperature of 38-42°C ((3a*R*,4*R*,6*R*,6a*R*)-6-(4-aminopyrrolo[2,1-*f*][1,2,4]triazin-7-yl)-6-cyano-2-oxotetrahydrofuro[3,4-*d*][1,3]dioxol-4-yl)isobutyrate (0.5 g, 1.29 mmol) in ethyl acetate (6 mL), add 48% hydrobromic acid (0.22 g, 1.29 mmol) dropwise at 38-42°C. After addition, continue stirring at 38-42°C for 30 minutes. Solid precipitates. Filter, wash the filter cake with 4 mL ethyl acetate, and dry the filter cake to obtain the white solid compound (337 mg, 55.8%).

The obtained solid was a crystalline form. Its X-ray powder diffraction (XRPD) results are shown in Figure 6, which are substantially consistent with Crystal Form A in Figure 1.

### Example 9: Comparison of physicochemical properties and preparation of Compound II and its salts

### (1) Comparison of properties and preparation of Compound II and its different salts

Select Compound II and its different salts (prepared in Examples 1-7), compare their properties and ease of preparation. The results are shown in Table 1.

**Table 1 Properties and Preparation of Compound II and Its Salts**

| **Compound** | **Properties** | **Preparation Situation** |
|---|---|---|
| Compound II | White gum solid | / |
| Compound II hydrochloride | White powdery solid | Simple operation, easy filtration |
| Compound II hemisulfate | White powdery solid, needs grinding | System is gummy, difficult filtration |
| Compound II phosphate | White powdery solid, needs grinding | System is gummy, difficult filtration |
| Compound II nitrate | White powdery solid | Simple operation, easy filtration |
| Compound II maleate | White powdery solid | Simple operation, relatively difficult filtration |
| Compound II acetate | No product obtained | / |
| Compound II hydrobromide (Crystal Form A) | White crystalline solid | Simple operation, very easy filtration |
| Compound II hydrobromide (Crystal Form B) | White crystalline solid | Simple operation, very easy filtration |

As can be seen from Table 1 above, except for the acetate, the other acid addition salt forms of Compound II have good properties. Furthermore, the hydrobromide, hydrochloride, and nitrate salts of Compound II are easier to prepare, with the hydrobromide salt particularly presenting as a distinct white needle-like crystalline solid.

### (2) Comparison of hygroscopicity and stability of the salts of Compound II

Select different salts of Compound II (prepared in Examples 1-7), compare their hygroscopicity and stability. High temperature condition was (80°C), accelerated condition was (40°C/75% RH), long-term condition was (25°C/60% RH). Stability results are shown in Table 2, hygroscopicity and stability evaluation results are shown in Table 3.

**Table 2 Stability Data of Compound II and Salts of Compound II (HPLC Purity %)**

| **Compound** | **0 Day** | **Accelerated 7 Days (40°C /75%RH)** | **High Temp 7 Days (80°C)** | **Long Term 7 Days (25°C /60%RH)** |
|---|---|---|---|---|
| Compound II | 99.47 | 96.40 | 97.51 | 97.46 |
| Compound II hydrochloride | 99.76 | 96.85 | 99.66 | 99.72 |
| Compound II phosphate | 99.42 | 56.17 | 76.99 | 97.21 |
| Compound II nitrate | 99.85 | 97.25 | 97.73 | 97.82 |
| Compound II hemisulfate | 99.26 | 37.53 | 96.89 | 97.16 |
| Compound II maleate | 99.60 | 96.66 | 97.69 | 97.42 |
| Compound II hydrobromide (Crystal Form A) | 99.78 | 99.76 | 99.75 | 99.70 |
| Compound II hydrobromide (Crystal Form B) | 99.30 | 99.23 | 99.13 | 99.25 |

As can be seen from Table 2 above, the hydrobromide salts of Compound II (Crystal Form A and Crystal Form B) are very stable under high temperature, long-term, and accelerated conditions. The stability of Compound II hydrochloride is average; while the stability of Compound II hemisulfate, phosphate, nitrate, and maleate is relatively poor.

**Table 3 Hygroscopicity and Stability of Salts of Compound II**

| **Compound** | **Hygroscopicity** | **Stability** |
|---|---|---|
| Compound II hydrochloride | Hygroscopic, moisture increase 7.5% | Degradation after 7 days under high temp, long-term, accelerated conditions |
| Compound II hemisulfate | Hygroscopic, moisture increase 7.0% | Degradation after 7 days under high temp, long-term, accelerated conditions |
| Compound II phosphate | Highly hygroscopic, moisture increase 15.8% | Degradation after 7 days under high temp, long-term, accelerated conditions |
| Compound II nitrate | Hygroscopic, moisture increase 8.1% | Slight degradation after 7 days under accelerated conditions |
| Compound II maleate | Hygroscopic, moisture increase 6.0% | Degradation after 7 days under accelerated conditions |
| Compound II hydrobromide (Crystal Form A) | None, no moisture increase | Good stability after 7 days under high temp, long-term, accelerated conditions |
| Compound II hydrobromide (Crystal Form B) | None, moisture increase <0.2% | Good stability after 7 days under high temp, long-term, accelerated conditions |

As can be seen from Table 3 above, the hydrobromide salts of Compound II (Crystal Form A and Crystal Form B) are non-hygroscopic under conditions of 25°C, RH 80%; the hydrochloride, hemisulfate, phosphate, nitrate, and maleate salts of Compound II have significant hygroscopicity.

### (3) Comparison of solubility of Compound II and its salts

Select Compound II and its different salts (prepared in Examples 1-7), compare their solubility. The results are shown in Table 4.

**Table 4 Solubility of Salts of Compound II in Deionized Water**

| **Compound** | **Solubility in Water (mg/mL)** |
|---|---|
| Compound II | 0.033 |
| Compound II hydrochloride | 0.209 |
| Compound II hemisulfate | 0.218 |
| Compound II phosphate | 0.222 |
| Compound II nitrate | 0.105 |
| Compound II maleate | 0.156 |
| Compound II hydrobromide (Crystal Form A) | 0.394 |
| Compound II hydrobromide (Crystal Form B) | 0.382 |

From the table above, it can be seen that salt formation significantly increases the water solubility of Compound II. The water solubility and chemical stability of the compound have important impacts on pharmaceutical formulation and oral bioavailability. Therefore, converting Compound II into a salt can significantly improve its water solubility, which will be more conducive to drug development.

### Example 10: Determination of the composition ratio of the hydrobromide salt of Compound II

Ion chromatography was used to determine the composition ratio of the hydrobromide salt of Compound II. The results are shown in Table 5.

### Table 5 Composition Ratio of the Hydrobromide Salt of Compound II

| **Hydrobromide Salt of Compound II** | **Bromide Ion Content (%) Measured** | **Composition Ratio** | **Bromide Ion Content (%) Theoretical** |
|---|---|---|---|
| Crystal Form A | 16.90 | 1:1 | 17.06 |
| Crystal Form B | 16.95 | 1:1 | |

As can be seen from Table 5 above, Compound II and hydrogen bromide form a salt in a molar ratio of 1:1.

### Test Example 1: Cytotoxic effects of Compound II, Compound II hydrobromide (Crystal Form A), Compound II hydrochloride, GS-441524, and Ribavirin on HEp-2 cells.

HEp-2 cells were seeded at 6,000 cells per well, 100 µL per well, in 96-well plates and cultured overnight in a 37°C, 5% CO₂ incubator. The next day, add 50 µL of gradient diluted test compounds (Compound II, Compound II hydrobromide (Crystal Form A), Compound II hydrochloride, and GS-441524 at concentrations of 50 µM, 16.667 µM, 5.556 µM, 1.852 µM, 0.617 µM, 0.206 µM, 0.069 µM, 0.023 µM; positive control Ribavirin at corresponding concentrations of 300 µM, 100 µM, 33.333 µM, 11.111 µM, 3.704 µM, 1.235 µM, 0.412 µM, 0.137 µM) (8 concentration points, 3-fold serial dilution, triplicate wells). Set up a blank cell control group (normal cells, no compound treatment). The final concentration of DMSO in the cell culture medium was 0.5%. The cells were placed in a 37°C, 5% CO₂ incubator and cultured for 5 days. Cell viability was detected using CCK-8 reagent, and cell survival rate was calculated. The results showed that Compound II, Compound II hydrobromide (Crystal Form A), Compound II hydrochloride, and GS-441524 had no cytotoxicity against HEp-2 cells at 50 µM, indicating good safety.

### Test Example 2: In vitro anti-respiratory syncytial virus (RSV) A2 strain activity of Compound II, Compound II hydrobromide (Crystal Form A), Compound II hydrochloride, GS-441524, and Ribavirin.

HEp-2 cells were seeded at 6000 cells per well, 100 µL per well, in 96-well plates and cultured overnight in a 37°C, 5% CO₂ incubator. The next day, add 50 µL of gradient diluted compounds (8 concentration points same as Test 1, 3-fold serial dilution, triplicate wells) and 50 µL of virus (200 TCID₅₀). Set up cell control (normal cells, no virus infection or compound treatment) and virus control (cells infected with virus, no compound treatment). The final concentration of DMSO in the cell culture medium was 0.5%. The cells were placed in a 37°C, 5% CO₂ incubator and cultured for 5 days.

Virus F protein expression in each well was detected using ELISA. After 5 days of infection, discard the supernatant, add 75 µL of 80% acetone to each well to fix the cells. After removing acetone, add 75 µL of rabbit anti-RSV F protein antibody to each well, incubate at 37°C for 1 h, wash the plate three times with TBST. Add 75 µL of HRP-labeled goat anti-rabbit IgG antibody to each well, incubate at 37°C for 1 h, wash the plate three times with TBST. Add 100 µL of TMB chromogenic solution to each well, react at room temperature for 20-30 minutes, then add 100 µL of 1% HCl to each well to stop the color reaction. After shaking evenly, read the absorbance value (OD₄₅₀) of each well using a microplate reader. The antiviral activity of the compounds was calculated from the obtained raw data. The selectivity index (SI) was calculated using the formula "SI = CC₅₀ / EC₅₀". The antiviral activity test results are shown in Table 6.

**Table 6 Cytotoxicity and Anti-RSV A2 Activity Results of All Compounds**

| **Compound** | **EC₅₀ (µM)** | **CC₅₀ (µM)** | **SI** |
|---|---|---|---|
| Compound II | 0.599 | >50 | >83.51 |
| Compound II hydrobromide (Crystal Form A) | 0.579 | >50 | >86.42 |
| Compound II hydrochloride | 1.195 | >50 | >41.84 |
| GS-441524 | 1.278 | >50 | >39.12 |
| Ribavirin | 26.960 | 205.200 | 7.61 |

From Table 6, it can be seen that Compound II, Compound II hydrochloride, Compound II hydrobromide (Crystal Form A), and GS-441524 showed no cytotoxicity within the tested range, indicating good safety; Compound II, Compound II hydrochloride, Compound II hydrobromide (Crystal Form A), and GS-441524 all exhibited antiviral activity against the RSV A2 virus strain, and the inhibitory effect was significantly better than the positive control drug Ribavirin. Among them, the hydrobromide (Crystal Form A) salt had the best activity and the highest selectivity index.

### Test Example 3: Cytotoxic effects of Compound II, Compound II hydrobromide (Crystal Form A), Compound II hydrochloride, and GS-441524 on Vero-E6 cells.

Digest well-growing Vero-E6 cells with trypsin-EDTA, count; adjust cell concentration to 2×10⁵/mL with DMEM culture medium containing 10% fetal bovine serum; inoculate the above cell suspension into 96-well plates for culture, 100 µL per well, and form a monolayer after 24 h culture in 5% CO2, 37°C. After 24 h, discard the culture medium, wash with PBS, dry, and replace with new DMEM culture medium. Add compounds Compound II, Compound II hydrobromide (Crystal Form A), Compound II hydrochloride, and GS-441524 respectively, with final concentrations of 50 µM, 10 µM, 5 µM, 1 µM, 0.1 µM. Set up a blank cell control group at the same time. After 48 hours of culture, detect cell viability using CelITiter-Glo^{®} reagent, and calculate cell survival rate. The results showed that at final concentrations of 50 µM, 10 µM, 5 µM, 1 µM, 0.1 µM, all the above compounds had no cytotoxicity against Vero-E6 cells, indicating good safety.

### Test Example 4: In vitro anti-SARS-CoV-2 virus activity of Compound II, Compound II hydrobromide (Crystal Form A), Compound II hydrochloride, and GS-441524.

Cell preparation: Count Vero-E6 cells at 2×10⁵/ml, seed 100 µL cell suspension per well in 96-well plates, place in a 37°C, 5% CO₂ incubator overnight. When cells grow into a monolayer, they are ready for use.

Virus dilution: Add 0.9 mL of DMEM containing 5% fetal bovine serum to the first tube, and 1.8 mL to the remaining tubes; add 0.1 mL of virus stock solution to the first tube, then change to a new pipette tip and draw 0.2 mL from the first tube into the second tube; sequentially perform twofold serial dilution to the highest dilution.

Virus inoculation: Wash the cells once with serum-free DMEM in the 96-well plate with a CRFK cell monolayer; add 100 µL of different virus dilutions to each well, perform one column of wells for each dilution, i.e., 8 parallel replicate wells for each virus dilution. Start adding from the highest dilution. Set negative controls, add 100 µL of DMEM containing 5% fetal bovine serum to negative control wells to check cell survival. Culture in 5% CO₂, 37°C for 5 days. After 5 days, observe under microscope, count the number of wells showing CPE in each column, and calculate virus TCID₅₀/mL using the KARBER method.

Virus viability detection: Inoculate VeroE6 cells at 2×10⁴ cells/well in 96-well culture plates, culture at 37°C for 24 h to form a monolayer. The initial drug concentration for Compound II, Compound II hydrobromide (Crystal Form A), Compound II hydrochloride, and GS-441524 was 6.0 µM (solution in DMEM medium with 0.5% DMSO), inactivated and then diluted 1:2, 1:4, 1:8, 1:16, 1:32, 1:64, 1:128. Perform 3 parallel wells for each dilution. Then mix equal volumes of the above test drugs at various dilutions with 100 TCID₅₀ virus solution, let react at 37°C for 1 h, then inoculate into Vero E6 cells, culture at 37°C, 5% CO₂ for 5 days, observe the highest virus dilution causing 50% cytopathic effect (CPE). Set up control groups at the same time: including cell control (culture medium with cells only, no virus serum), negative control (culture medium containing 100 TCID₅₀ virus, cells, and blank serum), and blank control (culture medium containing 100 TCID₅₀ virus and cells, no serum). Based on the degree of CPE, calculate the 50% endpoint drug concentration using the Reed-Muench method, i.e., the drug dilution at which 50% of cells do not produce CPE. The test results are shown in Table 7.

**Table 7 Drug Dilution Causing CPE in 50% of Cells**

| No. | SARS-CoV-2 Variant | | |
|---|---|---|---|
| | BA.5 | BF.7 | XBB |
| Compound II | 1:128 | 1:128 | 1:128 |
| Compound II hydrobromide (Crystal Form A) | 1:128 | 1:128 | 1:128 |
| Compound II hydrochloride | 1:64 | 1:64 | 1:64 |
| GS-441524 | 1:24 | 1:24 | 1:24 |

The microplate method was used to evaluate the inhibitory effect of the drugs on the novel coronavirus (a higher dilution factor indicates higher antiviral activity). From Table 7, it can be seen that Compound II, Compound II hydrobromide (Crystal Form A), Compound II hydrochloride, and GS-441524 all have significant inhibitory effects on the three novel coronavirus variants (BA.5, BF.7, and XBB). The inhibitory effects of Compound II, Compound II hydrobromide (Crystal Form A), and Compound II hydrochloride are significantly better than that of GS-441524.

### Test Example 5: Cytotoxic effects of Compound II, Compound II hydrobromide (Crystal Form A), Compound II hydrochloride, and GS-441524 on CRFK cells.

Gradient dilution of drugs: Weigh Compound II, Compound II hydrobromide (Crystal Form A), Compound II hydrochloride, and GS-441524, dissolve them in DMSO respectively, then dilute GS-441524 with maintenance medium into 25 mM, 12.5 mM, 6.25 mM, 3.125 mM, 1.5625 mM, 0.390 mM, 0.098 mM solutions. Dilute all the above test drugs with maintenance medium into 16 mM, 8 mM, 4 mM, 2 mM, 1 mM, 0.25 mM, 0.0625 mM solutions, making the DMSO concentration 1.0%. Filter sterilize using a 0.22 µm filter, store at 4°C for later use.

Determination of drug cytotoxicity to cells: Pre-culture CRFK cells in complete medium in 96-well plates, place in a 37°C, 5% CO₂ incubator. When the cells grow into a monolayer, discard the culture medium in the wells. Add 0.1 mL of different dilutions of Compound II, Compound II hydrobromide (Crystal Form A), Compound II hydrochloride, and GS-441524 to the cell culture wells, with 6 replicate wells for each concentration. Set up cell control wells and blank control wells at the same time. After 48h of culture, add 10µL of CCK8 reagent to each well, continue incubating for 0.5h, then measure the absorbance value (OD₄₅₀) at 450nm wavelength using a microplate reader. Calculate the cell survival percentage based on the control group. Cell survival percentage (%) = (Average OD of test group - Average OD of blank control group) / (Average OD of cell control group - Average OD of blank control group) × 100%.

Results of drug cytotoxicity determination: Statistical analysis showed that when the concentration of Compound II was ≤1 mM, Compound II hydrobromide (Crystal Form A) was ≤1 mM, Compound II hydrochloride was ≤0.51 mM, and GS-441524 was ≤0.39 mM, the OD₄₅₀ values of each concentration drug group were not different from the cell control group (P > 0.05), indicating no significant inhibitory effect on CRFK cell growth. That is, the maximum safe concentrations of Compound II, Compound II hydrobromide (Crystal Form A), Compound II hydrochloride, and GS-441524 were 0.87 mM, 1.0 mM, 0.51 mM, and 0.39 mM, respectively.

### Test Example 6: In vitro anti-feline infectious peritonitis virus (FIPV) activity of Compound II, Compound II hydrobromide (Crystal Form A), Compound II hydrochloride, and GS-441524.

Virus dilution: Perform 10-fold serial dilution of the virus solution. First, aliquot blank DMEM medium into 10 tubes, 0.9 mL per tube. Take 0.1 mL of virus suspension and add it to the first tube, mix well to make 10⁻¹. Take 0.1 mL from the 10⁻¹ virus solution and put it into the second tube, mix well to make 10⁻². Dilute sequentially up to 10⁻⁷.

Virus inoculation: Discard the culture medium from the 96-well plate with a CRFK cell monolayer. Inoculate the diluted virus solution into the wells of the 96-well plate, 0.1 mL per well. Perform 6 replicates for each dilution, and set up normal cell controls. After inoculation is completed, place together in a 37°C, 5% CO₂ incubator for adsorption for 3 h. After adsorption is complete, discard the virus solution. Add 0.1 mL of maintenance medium (containing 2% FBS) to each well of cells. Place in a 37°C, 5% CO₂ incubator for culture. Observe the CPE results daily, record the number of wells with CPE, and calculate TCID₅₀/100 µL using the Reed-Muench method.

Virus viability detection: Add 100 TCID₅₀ of FIPV virus in 0.1 mL to each well, adsorb for 3 h, pour off the virus solution. Dilute Compound II, Compound II hydrobromide (Crystal Form A), Compound II hydrochloride, and GS-441524 with maintenance medium containing 0.1% DMSO to different compound concentrations of 5 µM, 2.5 µM, 1.25 µM, 0.625 µM, 0.5 µM, 0.3125 µM, 0.25 µM. Add 100 µL to each well, perform 5 replicates for each concentration. Set up virus control (add virus, no drug solution) and cell control (no virus, no drug solution) at the same time. Place in a 37°C, 5% CO₂ incubator for culture. Observe Cytopathic Effect (CPE) daily under microscope. When the CPE degree in the virus control wells reaches 100%, add CCK-8 reagent to detect the OD₄₅₀ value of each well. Calculate the virus replication inhibition rate of different compound concentrations, draw a curve, and calculate the half maximal effective concentration (EC₅₀). The test results are shown in Table 8.

**Table 8 Inhibitory Results of All Compounds against Feline Infectious Peritonitis Virus**

| **Compound** | **EC₅₀** |
|---|---|
| Compound II | 0.70 µM |
| Compound II hydrobromide (Crystal Form A) | 0.64 µM |
| Compound II hydrochloride | 0.75 µM |
| GS-441524 | 0.73 µM |

From Table 8, it can be seen that Compound II, Compound II hydrobromide (Crystal Form A), Compound II hydrochloride, and GS-441524 all have significant inhibitory effects on feline infectious peritonitis virus. Among them, Compound II hydrobromide (Crystal Form A) has the best inhibitory effect.

### Test Example 7: In vitro anti-feline calicivirus (FCV) activity of Compound II, Compound II hydrobromide (Crystal Form A), Compound II hydrochloride, and GS-441524.

Cell preparation: Count feline kidney (CRFK) cells at 1×10⁵/ml, seed 100 µL cell suspension per well in 96-well plates, place in a 37°C, 5% CO₂ incubator overnight. Divide into test groups (different drug concentrations), normal cell group (no virus and drug), virus group (no drug), with 3 replicate wells per group. Virus viability detection: Add 100 TCID₅₀ of feline calicivirus (FCV) in 100 µL to each well. After overnight adsorption, discard the supernatant, wash with PBS. Prepare a 400 µM drug solution, filter sterilize, and dilute Compound II, Compound II hydrobromide (Crystal Form A), Compound II hydrochloride, and GS-441524 with maintenance medium to different drug concentrations of 100 µM, 50 µM, 25 µM, 12.5 µM, 6.25 µM, 3.125 µM, 1.5625 µM, 0.78125 µM. Add 100 µL to each well. Wait until the CPE in the virus group reaches 75%-100%, then observe CPE. Score: 0%-25% CPE = 1, 25%-50% = 2, 50%-75% = 3, 75%-100% = 4. The results are shown in Table 9.

**Table 9 Percentage of Cells Showing CPE at Different Drug**

| **Drug Concentratio n (µM)** | **Compound Number** | | | |
|---|---|---|---|---|
| | **Compoun d II** | **Compound II Hydrobromid e (Crystal Form A)** | **Compound II Hydrochlorid e** | **GS-44152 4** |
| 100 | 1 | 1 | 1 | 1 |
| 50 | 1 | 1 | 1 | 1 |
| 25 | 1 | 1 | 1 | 2 |
| 12.5 | 2 | 1 | 2 | 2 |
| 6.25 | 2 | 2 | 2 | 3 |
| 3.125 | 3 | 2 | 3 | 3 |
| 1.5625 | 3 | 3 | 3 | 4 |
| 0.78125 | 4 | 4 | 4 | 4 |

The inhibitory effect of the drugs on feline calicivirus was evaluated by the cell cytopathic effect (CPE). From Table 9, it can be seen that at a drug concentration of 12.5 µM, all compounds have a significant inhibitory effect on feline calicivirus, with a CPE rate of less than 50%.

### Test Example 8: Pharmacokinetic evaluation in rats

Pharmacokinetic process: Take 21 SD male rats (weight 180-220 g), 3 per group. Fast for 12h before the experiment, allow free access to water, provide unified feeding 2h after administration. Dissolve Compound II, Compound II hydrobromide (Crystal Form A), and GS-441524 in appropriate solvents. The administration vehicle was DMSO/propylene glycol/0.9% NaCl solution. The administration dose was 0.103 µmol/kg for all, administered by oral gavage. After administration to each group of rats, collect 0.3-0.5 ml of blood from the orbital sinus at 0.25, 0.5, 1, 2, 3, 4, 8, 12 h respectively, place in 1.5 ml EP tubes (pretreated with heparin), centrifuge at 4°C, separate the upper plasma containing the drug, and freeze at -80°C for testing. The concentration of GS-441524 in plasma was determined by LC-MS-MS, and pharmacokinetic parameters were calculated.

Standard curve: Take about 5 ml of blank rat blood, centrifuge, separate plasma. Take 6 pieces of 1.5 ml EP tubes, add 0.1, 0.5, 1, 5, 10, 20, 50 µl of stock solution respectively, supplement with working solvent to 450 µl, mix well. Add 50 µl of blank plasma to each, vortex for 5 min, centrifuge for 5min, take the supernatant, centrifuge again for 5min, take the supernatant into 1.5ml EP tubes, to obtain GS-441524 standard curve samples at 10, 50, 100, 500, 1000, 2000, 5000 ng/ml (containing internal standard 6,7-Dimethyl-2,3-Bis(2-pyridyl)quinoxaline, QX 100 ng/ml).

Test sample processing: Take 50 µl of the drug-containing plasma obtained from the pharmacokinetic process respectively into 1.5 ml EP tubes containing 450 µl of working solvent, vortex for 5 min, take the supernatant, centrifuge again for 5 min, take the supernatant into 1.5 ml EP tubes, to obtain the test compound pharmacokinetic detection samples.

LC-MS detection: Chromatographic column, Kinetex C18 core-shell universal column (Phenomenex, USA), column temperature 30°C, injection volume 10 µL, mobile phase acetonitrile-0.6% ammonium formate, flow rate 1.0 ml/min, isocratic elution; ion source, electrospray ion source (ESI); detection mode, multiple reaction monitoring (MRM); scanning mode, positive ion mode; ESI spray voltage, ion source temperature 400°C, nebulizer gas flow rate 150 µL/min, drying gas flow rate 100 µL/min.

The pharmacokinetic results are detailed in Table 10.

**Table 10 Pharmacokinetic Parameter Results of All Compounds (Mean ± SD)**

| **Compound** | **GS-441524** | **Compound II** | **Compound II Hydrobromide (Crystal Form A)** |
|---|---|---|---|
| Administration Route | ig | ig | ig |
| T_{1/2} (h) | 1.49 ± 0.45 | 1.31 ± 0.37 | 2.31 ± 0.48 |
| Tₘₐₓ (h) | 1.45 ± 0.61 | 0.81 ± 0.55 | 0.63 ± 0.47 |
| Cₘₐₓ (ng·mL⁻¹) | 904 ± 412.3 | 2038.5 ± 415.7 | 3708 ± 1031.0 |
| AUC₀₋ₜ (h·ng·mL⁻¹) | 2684.5 ± 538.9 | 5653.4 ± 752.6 | 11183.3 ± 1650.6 |
| AUC_{0-∞}(h·ng·mL⁻¹) | 2773 ± 878.4 | 7751.9 ± 1135.2 | 12382.2 ± 1449.7 |

### Test Example 9: Toxicity test of a single oral administration of Compound II hydrobromide (Crystal Form A) to SD rats.

Method: The test used a total of 50 SD rats (25/sex), randomly divided into 5 groups according to body weight, 10 rats per group (5/sex/group). Administer a single oral dose of vehicle (0.5% sodium carboxymethyl cellulose aqueous solution) and Compound II hydrobromide (Crystal Form A) dissolved in this vehicle (Groups 1-5: 5000, 3344, 2236, 1495, and 1000 mg/kg, respectively). The administration volume was 10 mL/kg. After administration, observe continuously for 14 days. All animals were dissected on D15. During the test, conduct clinical observations, body weight, food consumption, gross anatomy, and histopathological examination of abnormal organs.

Results: During the test, no deaths/moribundity were observed in male animals of each administration group and female animals in the 1000 mg/kg dose group. Female animals at doses ≥1000 mg/kg showed abnormal appearance (red urine color); a total of 7 female animals died in the ≥1495 mg/kg dose groups. Female animals showed excessive salivation, eye discharge, emaciation, lethargy, hunched back, prone position, reduced spontaneous activity, nasal discharge, general fluffy fur, and/or tiptoe gait, reduced food consumption. Male animals showed excessive salivation and eye discharge. Except for the dead animals, no test article-related abnormal changes were observed in body weight and weight gain of animals in each administration group.

In summary, the response to the test article differed significantly between male and female animals. The acute oral LD₅₀ of this test article for female rats was about 3481 mg/kg, with a 95% confidence limit for LD₅₀ of 2539-4772 mg/kg, belonging to low toxicity; no deaths were observed in male rats, belonging to practically non-toxic.

### Test Example 10: Dose range-finding test of repeated oral administration of Compound II hydrobromide (Crystal Form A) to SD rats for 30 days.

Method: The test used a total of 64 SD rats (32/sex), randomly divided into 7 groups according to body weight. Groups 1-4 were for toxicology study (5/sex/group), Groups 5-7 (4/sex/group) were for toxicokinetic study. Group 1 animals were given 0.5% sodium carboxymethyl cellulose aqueous solution as the vehicle control group. Groups 2 and 5, Groups 3 and 6, Groups 4 and 7 were given the vehicle 0.5% sodium carboxymethyl cellulose aqueous solution and Compound II hydrobromide (Crystal Form A) dissolved in this vehicle (30, 100, and 300 mg/kg, respectively). Animals were administered by oral gavage, administration volume 10 mL/kg, once daily for 30 consecutive days. Animals in Groups 1-4 were dissected the day after the last dose (D31). Animals in the toxicokinetic study groups were euthanized the day after the last dose (D31), without dissection.

During the test, conduct clinical observations, body weight, food consumption, body temperature, ophthalmological examination, clinical pathology, gross anatomy, organ weight, and histopathological examination. Also, determine the content of Compound II hydrobromide (Crystal Form A) and its metabolite GS441524 in samples from toxicokinetic study animals on D1 and D30, and perform toxicokinetic analysis.

Results: During the test, no deaths or moribundity were observed in all animals. Clinical observations of 30 mg/kg animals showed no test article-related abnormal changes. Ophthalmological examination, urinalysis, and gross anatomy of 100 mg/kg and 300 mg/kg group animals showed no test article-related abnormal changes. The main changes included: animals in the 100 mg/kg dose group showed excessive salivation; animals in the 300 mg/kg dose group showed excessive salivation, decreased body weight and food consumption, slight increase in hemoglobin (HGB), etc.

In summary, under the conditions of this test, Compound II hydrobromide (Crystal Form A) was administered orally to SD rats at doses of 30, 100, and 300 mg/kg, once daily for 30 consecutive days. The maximum tolerated dose (MTD) was 300 mg/kg. At this dose, the average peak concentration (Cₘₐₓ) and area under the curve AUCₗₐₛₜ of GS441524 in male animals on D30 were 7398.30 ng/mL and 41061.39 h·ng/mL, respectively, and in female animals were 9549.49 ng/mL and 36641.16 h·ng/mL, respectively.

Finally, it should be noted that the above embodiments are only used to illustrate the technical solutions of the present invention and not to limit them; although the present invention has been described in detail with reference to the foregoing embodiments, those of ordinary skill in the art should understand that they can still modify the technical solutions described in the foregoing embodiments or make equivalent substitutions for some of the technical features; and these modifications or substitutions do not make the essence of the corresponding technical solutions deviate from the spirit and scope of the technical solutions of the embodiments of the present invention.

### Industrial Applicability

The present invention provides a salt of a cyclic carbonate nucleoside compound, its crystal forms, preparation methods, and applications. The salt of the cyclic carbonate nucleoside compound has the structure shown in Formula I. When Y is hydrobromic acid and n=1, the salt of this nucleoside compound exists as Crystal Form A or Crystal Form B. Crystal Form A has advantages such as good physical and chemical stability, good solid properties, good water solubility, low hygroscopicity, and high oral bioavailability. It can be used to prepare drugs for treating and/or alleviating related diseases caused by viruses (particularly novel coronavirus, feline infectious peritonitis virus, respiratory syncytial virus, porcine epidemic diarrhea virus, feline calicivirus, etc.), and has good economic value and application prospects.

## Claims

1. A salt of a cyclic carbonate nucleoside compound, having the structure represented by Formula I: 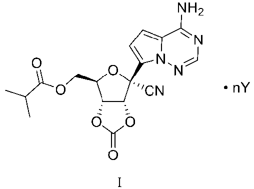
Wherein Y is hydrochloric acid, sulfuric acid, nitric acid, phosphoric acid, hydrobromic acid, maleic acid, succinic acid, tartaric acid, hydroiodic acid, fumaric acid, benzoic acid, phthalic acid, methanesulfonic acid, benzenesulfonic acid, p-toluenesulfonic acid, camphoric acid, camphorsulfonic acid, salicylic acid, trifluoroacetic acid, acetic acid, perchloric acid, malic acid, formic acid, propionic acid, malonic acid, oxalic acid, naphthalenesulfonic acid, acetylsalicylic acid, aspartic acid, glutamic acid, lactic acid, gluconic acid, ascorbic acid, gallic acid, mandelic acid, sorbic acid, taurine, homotaurine, cinnamic acid, mucic acid, or a combination thereof, preferably hydrobromic acid or hydrochloric acid;
n is 0.5-2, preferably 1.

2. A salt of a cyclic carbonate nucleoside compound, **characterized in that** its structural formula is represented by Formula I-1: 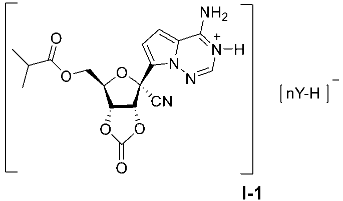
Wherein Y is hydrochloric acid, sulfuric acid, nitric acid, phosphoric acid, hydrobromic acid, maleic acid, succinic acid, tartaric acid, hydroiodic acid, fumaric acid, benzoic acid, phthalic acid, methanesulfonic acid, benzenesulfonic acid, p-toluenesulfonic acid, camphoric acid, camphorsulfonic acid, salicylic acid, trifluoroacetic acid, acetic acid, perchloric acid, malic acid, formic acid, propionic acid, malonic acid, oxalic acid, naphthalenesulfonic acid, acetylsalicylic acid, aspartic acid, glutamic acid, lactic acid, gluconic acid, ascorbic acid, gallic acid, mandelic acid, sorbic acid, taurine, homotaurine, cinnamic acid, mucic acid, or a combination thereof, preferably hydrobromic acid or hydrochloric acid;
n is 0.5-2, preferably 1.

3. A hydrobromide crystal of a cyclic carbonate nucleoside compound, **characterized in that** its structural formula is represented by Formula I-1': 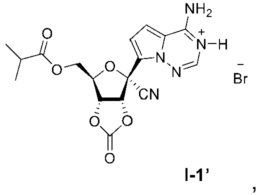 said crystal having Crystal Form A or Crystal Form B; wherein said Crystal Form A has at least one of the following characteristics:
Its X-ray powder diffraction pattern has characteristic peaks at at least 3, preferably at least 5, more preferably at least 7 of the following 2θ values: 5.42±0.2°, 10.87±0.2°, 16.56±0.2°, 18.88±0.2°, 21.83±0.2°, 23.81±0.2°, and 27.39±0.2°;
Further preferably, the X-ray powder diffraction pattern of said Crystal Form A also has characteristic peaks at at least 3, preferably at least 5, more preferably at least 8 of the following 2θ values: 25.09±0.2°, 27.02±0.2°, 28.26±0.2°, 29.05±0.2°, 29.84±0.2°, 31.71±0.2°, 33.52±0.2°, and 37.17±0.2°;
Its DSC spectrum has an absorption peak at 217±5°C;
The X-ray powder diffraction pattern of said Crystal Form B has characteristic peaks at at least 3, preferably at least 5, more preferably at least 8 of the following 2θ values: 5.33±0.2°, 11.07±0.2°, 19.49±0.2°, 20.19±0.2°, 21.04±0.2°, 25.50±0.2°, 30.31±0.2°, and 31.47±0.2°;
Further preferably, the X-ray powder diffraction pattern of said Crystal Form B also has characteristic peaks at at least 3, preferably at least 5, more preferably at least 8 of the following 2θ values: 14.44±0.2°, 16.41±0.2°, 17.20±0.2°, 19.72±0.2°, 21.46±0.2°, 22.88±0.2°, 23.58±0.2°, and 24.26±0.2°.

4. The hydrobromide crystal of a cyclic carbonate nucleoside compound according to claim 3, **characterized in that** the X-ray powder diffraction pattern of said Crystal Form A is substantially as shown in Figure 1;
The X-ray powder diffraction pattern of said Crystal Form B is substantially as shown in Figure 5.

5. A preparation method for the hydrobromide crystal of a cyclic carbonate nucleoside compound according to claim 3 or 4, **characterized in that** said crystal has Crystal Form A, said preparation method comprising the following steps:
Dissolving a compound of Formula II in Solvent A to obtain Solution A, mixing a solution of hydrobromic acid or hydrobromic acid dissolved in Solvent B (Solution B) with said Solution A and stirring to obtain a mixed solution, continuing to stir the mixed solution until solid precipitates, filtering, washing the filter cake with an appropriate amount of Solvent A, and drying to obtain the target product; 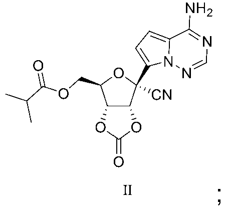
Further,
The usage ratio of said compound of Formula II to said Solvent A is 1 g : 2-20 mL, preferably 10-14 mL;
The temperature for preparing the mixed solution and waiting for solid precipitation is -15-50°C, preferably -5-15°C;
The molar ratio of said compound of Formula II to said hydrobromic acid is 1 : 0.5-2, preferably 1 : 1;
The stirring time is 0.5-12 hours, preferably 0.5-4 hours;
The usage ratio of said compound of Formula II to the Solvent A for washing the filter cake is 1 g : 4-20 mL, preferably 1 g : 8-12 mL;
Said Solvent A and Solvent B are each independently selected from ethyl acetate, acetonitrile, acetone, water, and homogeneous mixtures thereof, with Solvent A and Solvent B both preferably being ethyl acetate.

6. A preparation method for the hydrobromide crystal of a cyclic carbonate nucleoside compound according to claim 3 or 4, **characterized in that** said crystal has Crystal Form B, said preparation method comprising the following steps:
Dissolving a compound of Formula II in Solvent A to obtain Solution A, mixing a solution of hydrobromic acid or hydrobromic acid dissolved in Solvent B (Solution B) with said Solution A and stirring to obtain a mixed solution, continuing to stir the mixed solution, with no solid precipitation, concentrating the mixed solution under reduced pressure to obtain a gum; adding Solvent C to the gum, stirring until a large amount of solid precipitates, filtering, washing the filter cake with an appropriate amount of Solvent C, and drying to obtain the target product.

7. The preparation method according to claim 6, **characterized in that**,
The usage ratio of said compound of Formula II to said Solvent A is 1 g : 1-5 mL, preferably 1 g : 2-3 mL;
The amount of Solvent B used to dilute the acid is 1-4 times, preferably 2-3 times, the volume of the acid;
The stirring time is 10-60 minutes, preferably 30 minutes;
The usage ratio of said compound of Formula II to the washing Solvent C is 1 g : 4-20 mL, preferably 1 g : 8-12 mL;
Said Solvent A, Solvent B, and Solvent C are each independently selected from ethyl acetate, acetonitrile, acetone, water, and homogeneous mixtures thereof, with Solvent A and Solvent B both preferably being acetonitrile, and Solvent C preferably being ethyl acetate.

8. A pharmaceutical composition comprising the salt of the cyclic carbonate nucleoside compound according to claim 1 or 2, or the hydrobromide crystal of the cyclic carbonate nucleoside compound according to claim 3 or 4.

9. Use of the salt of the cyclic carbonate nucleoside compound according to claim 1 or 2, or the hydrobromide crystal of the cyclic carbonate nucleoside compound according to claim 3 or 4, or the pharmaceutical composition according to claim 8, in the treatment of diseases caused by infectious viruses; Preferably, said infectious viruses include one or more of novel coronavirus, feline infectious peritonitis virus, respiratory syncytial virus, porcine epidemic diarrhea virus, and feline calicivirus.

10. Use of the salt of the cyclic carbonate nucleoside compound according to claim 1 or 2, or the hydrobromide crystal of the cyclic carbonate nucleoside compound according to claim 3 or 4, or the pharmaceutical composition according to claim 8, in the manufacture of a medicament for treating diseases caused by infectious viruses;
Preferably, said infectious viruses include one or more of novel coronavirus, feline infectious peritonitis virus, respiratory syncytial virus, porcine epidemic diarrhea virus, and feline calicivirus.
